# FASCICULE DE BREVET EUROPEEN

(11) **EP 4 408 288 B1**
(45) Date de publication et mention de la délivrance du brevet: **12.02.2025**
(21) Numéro de dépôt: 22798272.5
(22) Date de dépôt: 29.09.2022
(51) Int. Cl.: A61B 5/22, A61B 5/391, A61B 5/00, A61N 1/05, A61N 1/36

(54) **SONDE PÉRINÉALE**
DAMMSONDE
PERINEAL PROBE

(30) Priorité: 30.09.2021 FR 2110352
(43) Date de publication de la demande: 07.08.2024
(73) Titulaire: Appareils pour Kinés Sports et Esthétique, 34470 Pérols (FR)
(72) Inventeur: BILLARD, Georges Robert, 34470 Pérols (FR)
(74) Mandataire: BARRE LAFORGUE
(86) Numéro de dépôt international: PCT/EP2022/077232
(87) Numéro de publication internationale: WO 2023/052567

(56) Documents cités:
- WO-A1-2016/202697
- CN-A- 113 274 028
- FR-A1- 2 827 520
- FR-A1- 2 930 713
- US-A1- 2013 018 281
- US-A1- 2018 055 563
- US-A1- 2019 160 332

## Description

### [Domaine technique]

L'invention concerne une sonde périnéale, et plus particulièrement, une sonde de diagnostic et de rééducation du périnée. Plus largement, l'invention s'inscrit dans le domaine paramédical et médical, l'invention vise, en particulier, à analyser l'état fonctionnel de structures anatomiques impliquées dans le mécanisme de la continence et/ou de l'incontinence urinaire et/ou fécale.

La continence est un mécanisme complexe qui implique un réflexe d'anticipation acquis et un réflexe myotatique inné qui en réponse à un stimulus sollicitent plusieurs groupes de muscles. Généralement, le stimulus correspond à une augmentation de la pression intra-abdominale. En réponse à ce stimulus, le réflexe d'anticipation et le réflexe myotatique entrainent la contraction de groupes de muscles en vue d'éviter une fuite urinaire et/ou fécale.

Ainsi, un premier groupe de muscles comprend les sphincters urétraux et anaux qui ceinturent respectivement l'urètre et le canal anal. Sous l'effet de leur contraction respective l'urètre et le canal anal se ferment. Les muscles du périnée constituent un deuxième groupe de muscles qui participe au mécanisme de la continence. Le périnée est un ensemble de muscles qui s'étendent du pubis au coccyx. De manière générale, le périnée exerce deux fonctions, d'une part, il maintient le contenu abdominal : rectum, vessie, utérus, et d'autre part, il participe à l'amortissement des hyperpressions abdominales et à la fermeture de la vessie et du rectum. De fait, le périnée est connu pour jouer un rôle important dans le mécanisme de la continence.

Au regard de ces fonctions physiologiques, les thérapeutes et les industrielles se sont intéressés au périnée pour diagnostiquer et traiter les troubles de l'incontinence tant chez les patients masculins que chez les patients féminins.

### [État de la technique antérieure]

D'un point de vue des thérapeutes, la première méthode d'appréciation de l'état fonctionnel du périnée, qui est encore utilisée à ce jour, est une méthode manuelle qui implique un touché vaginale ou rectale. Au cours de cet acte médical, le thérapeute évalue la contraction initiée par le ou la patiente. Selon cette méthode, l'évaluation de l'état fonctionnel du périnée est réalisée selon des critères subjectifs qui sont fortement corrélés avec l'expérience personnelle du thérapeute. Dans ce contexte, tant le diagnostic que la rééducation fonctionnelle ne garantissent aucune régularité dans l'évaluation des dysfonctionnements et les résultats de la rééducation.

On connaît déjà des sondes périnéales pour l'analyse fonctionnelle et la rééducation des structures anatomiques impliquées dans le mécanisme de la continence ou de l'incontinence. Des appareils alternatifs ou similaires sont décrits dans US 2018/055563 A1, CN 113274028 A, FR 2827520 A1, US 2019/160332 A1 et US 2013/018281 A1.

La plupart des sondes périnéales connues de l'état de la technique comportent un corps de sonde de forme cylindrique qui comporte une extrémité libre arrondie. Le corps de sonde comporte deux électrodes annulaires disposées à distance déterminée l'une de l'autre. Ce type de sonde périnéale est conçu pour être introduite dans la cavité vaginale d'une patiente et/ou la cavité anale d'un patient. Selon leurs dimensions ce type de sondes peut procurer des sensations d'inconfort lors de l'introduction et du retrait de la sonde de l'endocavité vaginale ou anale. Par ailleurs, généralement ce type de sonde est conçu pour la rééducation par électrothérapie du tissu conjonctif qui entoure le vagin et/ou la cavité anale mais aussi les muscles entourant la cavité vaginale ou anale. Le caractère annulaire des électrodes ne permet toutefois pas d'isoler les muscles les uns des autres afin de cibler le diagnostic ou le travail d'électrothérapie. De plus, la forme cylindrique du corps de la sonde ne permet pas de maintenir ce type de sonde en position lors des examens ou du protocole de rééducation. En effet, ce type de sonde a tendance à sortir de l'endocavité lorsque la pression intraabdominale augmente, ce qui rend les examens ou la rééducation fastidieux. Dans ce contexte, il est difficile déterminer l'état fonctionnel des muscles qui entourent l'endocavité dans une situation orthostatique ou orthodynamique du quotidien du ou de la patiente qui est susceptible d'entrainer une fuite urinaire ou fécale.

Le document FR 2 941 860 décrit un autre exemple de sonde périnéale qui est conçue pour être insérée dans la cavité vaginale. Ce type de sonde permet de mesurer la tonicité du tissu conjonctif qui entoure la cavité vaginale. Cette sonde périnéale comporte deux branches articulées et complémentaires. Au moins une branche possède une extrémité recourbée qui vient recouvrir l'extrémité libre de l'autre branche afin de former une tête de sonde arrondie. Chaque branche porte un capteur de force afin de déterminer le mouvement pendulaire des branches par un différentiel de force mesurée entre capteur avant et arrière. Après insertion de la sonde dans la cavité vaginale, le thérapeute applique un écartement des branches disposées selon un axe antéro-postérieur. Lorsque l'on mesure une faible baisse de force sur le capteur avant, le tissu conjonctif est de bonne qualité et suit le déplacement des branches. A l'inverse, lorsque l'on mesure une importante baisse de la force mesurée, le tissu conjonctif est de mauvaise qualité et suit plus lentement le déplacement des branches de la sonde. La mauvaise qualité du tissu conjonctif est un facteur connu de l'incontinence. Lorsque le thérapeute obtient un tel diagnostic, il peut appliquer des techniques d'électrostimulation qui améliorent la trophicité et la tonicité des tissus conjonctifs.

Ce type de sonde n'est pas franchement confortable pour les patientes et doit être maintenu en position par le thérapeute au cours de l'examen. De plus, ce type de sonde ne permet pas de déterminer de manière différenciée le dysfonctionnement des structures anatomiques impliquées dans le mécanisme de verrouillage de l'urètre ou du canal anal.

L'invention vise à répondre aux inconvénients de l'état de la technique.

### [Exposé de l'invention]

L'invention vise en particulier à fournir une sonde périnéale limitant les sensations d'inconfort tant lors de son introduction dans l'endocavité vaginale ou anale du ou de la patiente que lors de son utilisation, ou encore lors du retrait de la sonde périnéale.

L'invention vise également à fournir une sonde périnéale endocavitaire qui est maintenue en position dans l'endocavité vaginale ou anale lors de son utilisation. En particulier, l'invention vise à fournir une sonde périnéale endocavitaire qui tient en position alors que le ou la patiente se tient debout pour effectuer des mouvements physiques qui sont susceptibles d'entrainer une fuite urinaire ou fécale.

L'invention vise aussi à fournir un outil de diagnostic et/ou de rééducation capable d'évaluer l'état fonctionnel et/ou de traiter de manière différenciée et dyssymétrique les plans profonds des plans superficiels du périnée.

Dans des modes de réalisation, l'invention vise à proposer une sonde périnéale qui peut s'adapter aux dimensions anatomiques d'un grand nombre de patientes ou de patients.

Dans cette optique, l'invention concerne une sonde périnéale comprenant un corps de sonde qui comporte :.
- une portion cylindrique configurée pour être positionnée au niveau de l'ouverture de l'endocavité vaginale ou anale d'un(e) patient(e), et
- une portion évasée qui s'étend depuis une extrémité de la portion cylindrique, la portion évasée s'étend de manière évasée depuis la portion cylindrique jusqu'à un sommet libre, la portion évasée étant élastiquement déformable entre une position repliée et une position déployée de façon à prendre appui sur les parois de l'endocavité, et
- des organes électroniques configurés pour interagir avec des muscles du périnée d'un(e) patient(e) les organes électroniques étant disposés sur la portion évasée et/ou sur la portion cylindrique.

La sonde périnéale selon l'invention se caractérise en ce que, , d'une part, la portion évasée comporte au moins une paroi latérale qui présente une face interne délimitant un volume interne conique de la portion évasée, et d'autre part, la portion évasée comporte au moins une gorge longitudinale qui s'étend selon un plan de symétrie de la sonde périnéale.

La conformation du corps de la sonde périnéale qui présente une partie basse cylindrique et une partie haute évasée permet de maintenir la sonde périnéale en position alors que le ou la patiente réalise des exercices ou mouvement dans lesquels ses fuites urinaires ou fécales surviennent au quotidien. Par ailleurs, le caractère déformable de la portion évasée permet de réduire le désagrément lié à l'introduction de la sonde dans l'endocavité mais aussi de plaquer la portion évasée contre les parois de l'endocavité une fois la sonde périnéale en position dans ladite endocavité. L'endocavité vaginale ou anale est naturellement collapsée, après son insertion dans l'endocavité, la portion évasée se déploie et prend appui sur les parois de l'endocavité, lesdites parois sont ainsi collabées contre les muscles périnéaux sous-jacents. En outre, la disposition des organes électroniques sur la partie basse et/ou sur la partie haute du corps de la sonde permet de cibler les muscles des plans profonds et/ou des plans superficiels du périnée. Le caractère creux de la portion évasée et la gorge longitudinale améliorent l'amplitude de déformation de la portion évasée.

Dans des modes de réalisation de l'invention,.. la gorge longitudinale s'étend selon un plan de symétrie sagittal médian de la sonde périnéale et divise ladite au moins une paroi latérale en deux demi-parois.

Dans des modes de réalisation de l'invention, la portion évasée comporte au moins une gorge qui s'étend transversalement par rapport au plan sagittal médian PSM de la sonde périnéale, ladite gorge s'étendant sur la face extérieure de la paroi latérale au niveau d'une base disposée à la jonction entre la portion évasée et la portion cylindrique. Ladite gorge diminue l'épaisseur de la paroi latérale au niveau de sa base afin d'augmenter l'amplitude de repliement de la portion évasée.

Dans des modes de réalisation de l'invention, la portion évasée peut comporter au moins une ouverture disposée selon un plan de symétrie de la sonde périnéale. L'ouverture améliore également l'amplitude de déformation de la sonde. Plus précisément, la portion évasée peut comporter :
- une ouverture latérale disposée selon un plan de symétrie de la sonde périnéale, et
- une ouverture sommitale disposée au niveau du sommet libre de la portion évasée.

Dans des modes de réalisation de l'invention, la sonde périnéale peut comporter un organe de maintien qui est mobile entre une position déployée et une position repliée, en position déployée, l'organe de maintien permet de maintenir la portion évasée en position dans l'endocavité du ou de la patient(e), et, en position repliée, l'organe de maintien permet l'introduction de la sonde périnéale dans l'endocavité. Les ouvertures latérale et sommitale de la portion évasée améliorent le repliement de l'organe de maintien qui se dispose, en position repliée, dans le volume interne de la portion évasée. Ceci confère une plus grande compacité de la tête de la sonde périnéale pour son introduction dans l'endocavité.

De manière plus précise, l'organe de maintien peut comporter une languette qui s'étend de manière saillante de la portion évasée vers une extrémité libre, la languette étant saillante selon un plan de symétrie de la sonde périnéale. Ainsi, la languette peut exercer un effort élastique contre la paroi de l'endocavité. L'effort élastique participe alors au maintien de la sonde périnéale au sein de l'endocavité. En particulier, la languette exerce une pression sur la paroi antérieure du vagin ou de rectum de manière à plaquer cette paroi contre les muscles périnéaux profonds. De manière complémentaire, la languette peut comporter à son extrémité libre une surface striée. Cette surface striée peut alors coopérer avec la paroi de l'endocavité qui dans le cas d'un vagin comporte également des stries.

En outre, l'organe de maintien peut être attaché à la portion cylindrique de manière détachable. Ceci permet de prévoir plusieurs tailles de languettes afin de s'adapter à l'anatomie du ou de la patiente. Par ailleurs, la languette peut être saillante, en position déployée, de la portion évasée selon un axe antéro-postérieur situé sur le plan. Selon cette disposition la languette exerce en position déployée un effort antéro-postérieur sur la paroi de l'endocavité vaginale ou anale qui contribue à maintenir la sonde périnéale en position notamment lorsque la ou le patient réalise des exercices physiques de mise en situation pour évaluer l'état fonctionnel des muscles du périnées.

Dans des modes de réalisation de l'invention, la portion évasée peut s'étendre selon un angle d'inclinaison α par rapport à une paroi latérale de la portion cylindrique, l'angle d'inclinaison α étant compris entre 7°et 40°. L'inclinaison de la portion évasée correspond à l'inclinaison naturelle antéro-postérieur du vagin. Cette inclinaison améliore le maintien de la sonde périnéale dans l'endocavité mais fait également office de détrompeur, la sonde périnéale ne peut alors être introduite dans l'endocavité que dans une seule orientation antéro-postérieure.

Dans des modes de réalisation de l'invention, la portion évasée comprend deux organes électroniques disposés de part et d'autre d'un plan de symétrie de la sonde périnéale. Cette disposition permet d'interagir de manière différenciée avec les muscles du plan profond du périnée qui sont situés de part et d'autre de l'endocavité.

Dans des modes de réalisation de l'invention, la portion cylindrique comprend deux organes électroniques disposés de part et d'autre d'un plan de symétrie de la sonde périnéale. Cette disposition permet d'interagir de manière différenciée avec les muscles du plan superficiel du périnée qui sont situés de part et d'autre de l'endocavité.

Plus précisément, la portion cylindrique et la portion évasée peuvent comporter respectivement des organes électroniques constitués par des électrodes EMG, les organes électroniques étant disposées de part et d'autre d'un plan sagittal médian PSM de la sonde périnéale. Ainsi, on peut mesurer l'activité électromyographique des muscles des plans superficiels et profonds du périnée de manière différenciée de part et d'autre de l'endocavité.

Dans des modes de réalisation de l'invention, les organes électroniques peuvent comporter deux capteurs de force, les organes électroniques étant disposés de façon antéro-postérieure de part et d'autre d'un plan transversal médian PTM de la sonde périnéale. On peut ainsi mesurer, de manière isolée, les forces antéro-postérieures qui s'appliquent sur l'extrémité inférieure de l'endocavité.

### [Description des dessins]

D'autres caractéristiques et avantages de l'invention apparaîtront encore à la lecture de la description qui va suivre. Celle-ci est purement illustrative et doit être lue en regard des dessins annexés sur lesquels :
[Fig. 1] est une représentation en perspective d'une sonde périnéale conforme de l'invention.
[Fig. 2] est une représentation d'une vue de haut de la portion évasée de la sonde périnéale de la figure 1.
[Fig. 3] est une représentation en perspective d'une vue avant de la sonde périnéale de la figure 1.
[Fig. 4] est une représentation d'une vue arrière de la sonde périnéale de la figure 1, la sonde périnéale étant, schématiquement, mise en situation dans une endocavité d'un(e) patient(e).
[Fig. 5] est une représentation d'une vue de côté de la sonde périnéale de la figure 1, deux languettes de dimensions différentes étant représentées à titre de comparaison, l'angle d'inclinaison de la portion évasée est également schématique représenté.
[Fig. 6] est une représentation d'une vue de face d'un mode de réalisation de la sonde périnéale conforme de l'invention, la sonde périnéale comportant des moyens de réception de la languette ménagés sur la paroi latérale de la portion cylindrique de la sonde périnéale.
[Fig. 7] et [Fig. 8] sont des représentations de vues latérales de deux organes de maintien de dimensions distinctes de la sonde périnéale de la figure 1.
[Fig. 9] est une représentation schématique d'un diagramme représentant des données de l'activité neuromusculaire que l'on peut mesurer à l'aide des organes électroniques d'une sonde périnéale conforme de l'invention.

### [Description des modes de réalisation]

La présente invention concerne une sonde périnéale 10. Cette sonde périnéale est conçue pour le diagnostic et la rééducation du périnée d'un(e) patient(e). La sonde périnéale 10 de l'invention est configurée pour être au moins partiellement introduite dans la cavité vaginale d'une patiente. Toutefois, la sonde périnéale 10 peut être également introduite dans la cavité anale d'un(e) patient(e). Ceci permet notamment de traiter les patients masculins souffrant d'incontinence fécale. Dans ce document, le terme endocavité désigne indifféremment la cavité vaginale et la cavité anale d'un(e) patient(e).

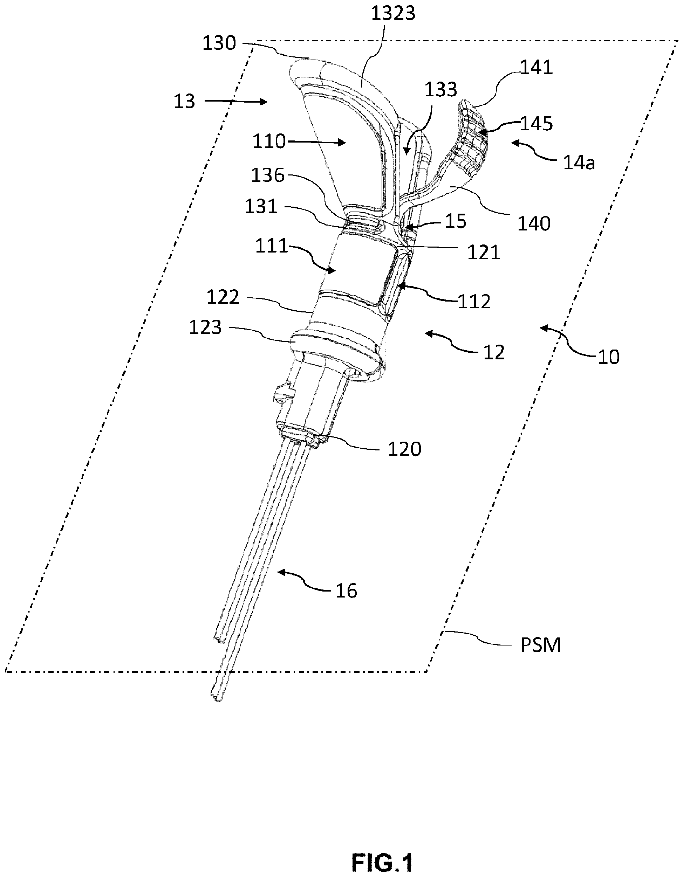

En référence aux figures 1 à 6, la sonde périnéale 10 comprend un corps de sonde qui comporte des organes électroniques 110, 111, 112 configurés pour interagir, au travers des parois de l'endocavité, avec des muscles du périnée d'un ou d'une patiente.

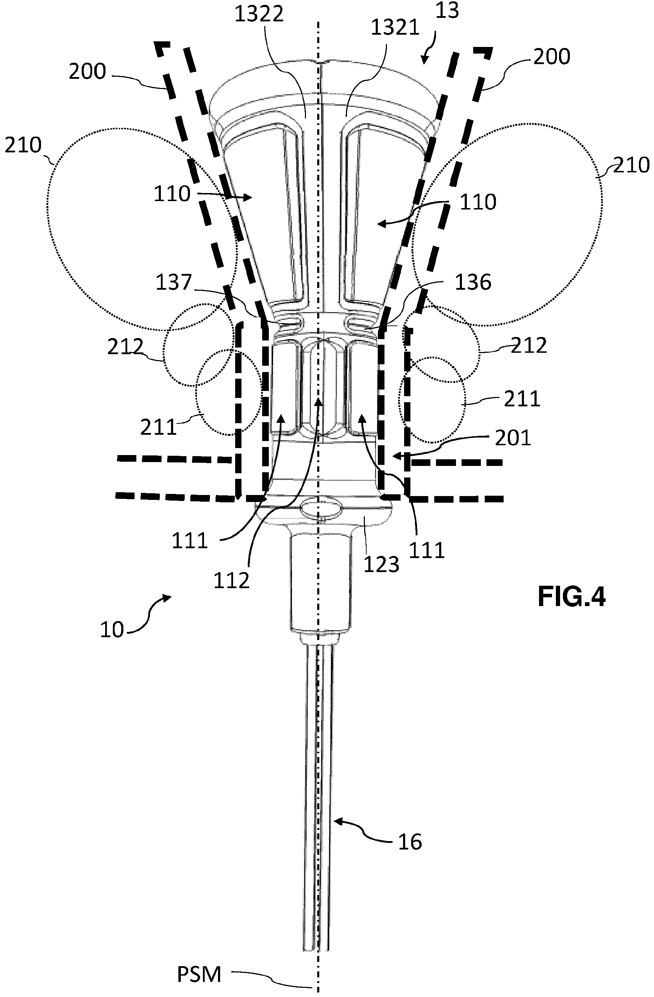

Comme cela est illustré à la figure 4, le corps de sonde comporte une portion cylindrique 12 configurée pour être positionnée au niveau de l'extrémité inférieure 201 de l'endocavité d'un ou d'une patiente. Dans cet exemple, le corps de sonde est réalisé dans un matériau polymérique médical tel que du silicone ou encore du polyuréthane. En particulier, le matériau polymérique sélectionné comporte des propriétés d'élasticité et d'étanchéité.

Comme illustré aux figures 1 à 6, la portion cylindrique 12 comprend une première extrémité 120 et une seconde extrémité 121 qui sont opposées l'une de l'autre. Ici, la première extrémité 120 de la portion cylindrique 12 est libre. Dans ce document, la première extrémité 120 définit l'extrémité inférieure de la sonde périnéale 10. Par convention, les termes « bas » et « inférieur » ainsi que leurs dérivés, lorsqu'ils sont utilisés spécifiquement en référence à la sonde périnéale 10, désignent la première extrémité 120 longitudinale de la portion cylindrique 12.

La seconde extrémité 121 de la portion cylindrique 12 comprend une paroi supérieure 124 qui délimite supérieurement la portion cylindrique 12.

Dans cet exemple, la portion cylindrique 12 comprend un premier tronçon et un second tronçon. La portion cylindrique 12 peut être équipée d'un bourrelet annulaire 123. Ici, le bourrelet annulaire 123 est disposé à la jonction entre le premier tronçon et le second tronçon de la portion cylindrique 12. Le bourrelet annulaire 123 permet d'épouser l'anatomie du ou de la patiente à l'ouverture de l'endocavité. Par exemple, pour un sujet féminin, lorsque la sonde périnéale 10 est introduite dans la cavité vaginale, le bourrelet annulaire 123 est placé au contact des petites lèvres de la vulve de la patiente. Ainsi, le premier tronçon s'étend longitudinalement entre le bourrelet annulaire 123 et la seconde extrémité 121 de la portion cylindrique 12 (illustré à la figure 4).

Comme illustré aux figures 1 et 3 à 6, le premier tronçon peut supporter des organes électroniques 111, 112. Au niveau du premier tronçon, la portion cylindrique 12 est délimitée par une paroi latérale 122. Par ailleurs, le second tronçon de la portion cylindrique 12 s'étend du bourrelet annulaire 123 à la première extrémité 120 de la portion cylindrique 12. Le second tronçon comporte des dimensions inférieures aux dimensions du premier tronçon.

Le corps de la sonde périnéale 10 comporte également une portion évasée 13 qui s'étend depuis une extrémité 121 de la portion cylindrique 12 jusqu'à un sommet libre 130. Dans ce document, le sommet libre 130 de la portion évasée 13 définit une extrémité longitudinale supérieure de la sonde périnéale 10. Par convention, les termes « haut » et « supérieurs » ainsi que leurs dérivés, lorsqu'ils sont utilisés spécifiquement en référence à la sonde périnéale 10, désignent l'extrémité libre 130 de la portion évasée. Le corps de la sonde périnéale 10 peut mesurer entre 5 cm et 15 cm entre son extrémité basse et son extrémité haute

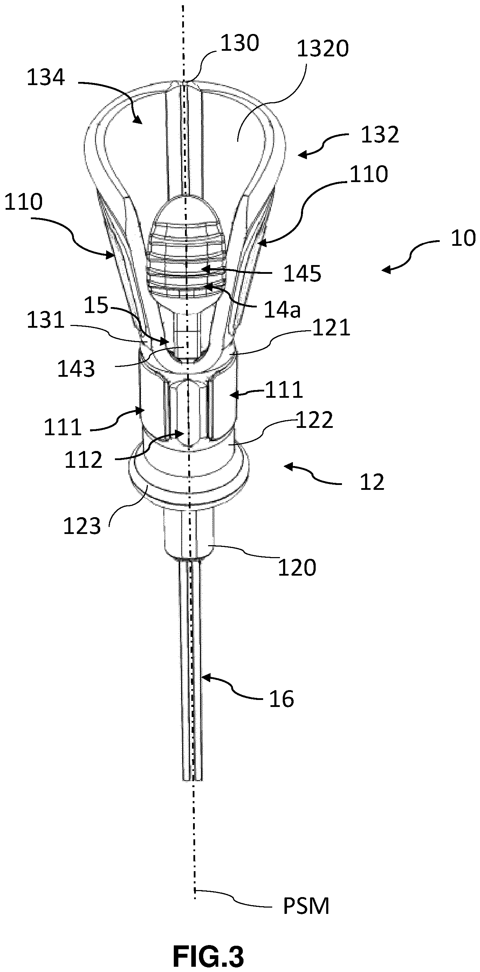

Comme illustré aux figures 3, 4 et 6, la portion évasée 13 s'étend de manière évasée depuis la portion cylindrique 12 jusqu'à un sommet libre 130 de la portion évasée 13. En particulier, la portion évasée 13 comprend une base 131 disposée à la jonction avec la seconde extrémité 121 de la portion cylindrique 12 du corps de sonde. Dans cet exemple, la portion évasée 13 s'étend depuis la base 131 jusqu'au sommet libre 130 de la portion évasée 13. Plus précisément, la base 131 du corps endocavitaire 13 est solidaire de la face supérieure 124 de la portion cylindrique 12. La portion évasée 13 constitue la tête de la sonde périnéale 10.

De plus, la portion évasée 13 est élastiquement déformable entre une position repliée et une position déployée. En position repliée, la tête de la sonde périnéale 10 occupe un volume plus petit qui facilite l'insertion de la sonde périnéale 10 dans l'endocavité. Le confort du ou de la patiente en est amélioré. Par ailleurs, en position déployée, la tête de la sonde peut prendre appui sur les parois de l'endocavité. De ce fait, la sonde périnéale 10 est maintenue dans l'endocavité d'un ou d'une patiente par la portion évasée 13 en position déployée. L'endocavité vaginale ou anale étant naturellement collapsée au repos, appliquer une pression sur les parois de l'endocavité permet de mettre en contact ces parois avec les structures anatomiques entourant l'endocavité telles que les muscles du périnée.

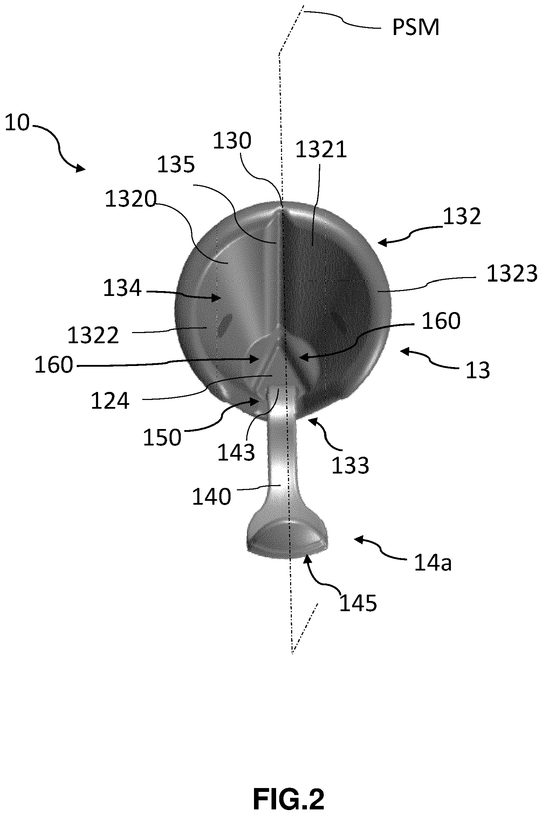

En particulier comme illustré aux figures 1 à 6, la portion évasée 13 comporte au moins une paroi latérale 132. Ici, la paroi latérale 132 s'étend de manière évasée depuis la base 131 vers le sommet libre 130 de la portion évasée 13. La paroi latérale 132 présente une face interne 1320 qui délimite un volume interne conique de la portion évasée 13 (illustré à la figure 2).

Dans cet exemple, la portion évasée 13 comporte au moins une ouverture 133, 134 disposée selon un plan de symétrie de la sonde périnéale 10. La ou les ouvertures facilitent la déformation élastique de la portion évasée 13 pour une introduction plus confortable de la sonde dans l'endocavité. En particulier, la portion évasée 13 comporte une ouverture latérale 133 et une ouverture sommitale 134.

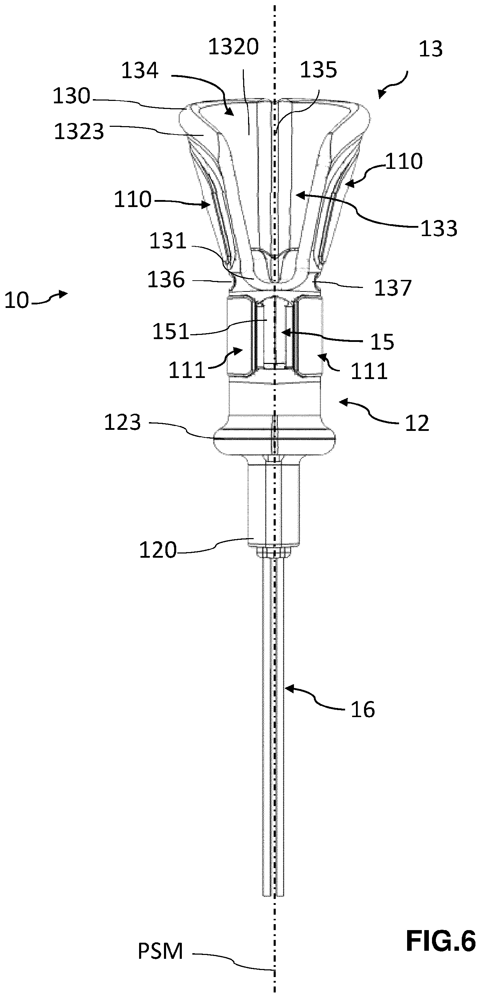

L'ouverture latérale 133 est disposée selon un plan de symétrie de la sonde périnéale 10. Comme illustré à la figure 6, l'ouverture latérale 133 s'étend longitudinalement de part et d'autre d'un plan sagittal médian PSM de la sonde périnéale 10. Le plan sagittal médian représenté aux figures 1 à 4 et 6 définit un plan de symétrie longitudinal selon une orientation sagittale de la sonde périnéale 10. L'ouverture latérale 133 s'étend depuis la base 131 de la tête jusqu'au somment libre 130 de la sonde périnéale 10. Comme illustré à la figure 6, l'ouverture latérale 133 présente une forme de U qui s'étend depuis la base 131, les branches du U s'écartent l'une par rapport à l'autre jusqu'au sommet 130 de manière à former une ouverture latérale 133 évasée.

Comme illustré notamment aux figures 2, 3 et 6, l'ouverture sommitale 134 est disposée au niveau du sommet libre 130 de la portion évasée 13. Comme illustré à la figure 2, l'ouverture sommitale 134 est symétrique par rapport au plan médian sagittal PSM de la sonde périnéale 10. La paroi latérale 133 comporte un bourrelet sommital 1323 qui délimite au moins partiellement l'ouverture sommitale 134. Le bourrelet sommital 1323 participe au confort du ou de la patiente lors de l'introduction de la sonde périnéale 10 dans l'endocavité.

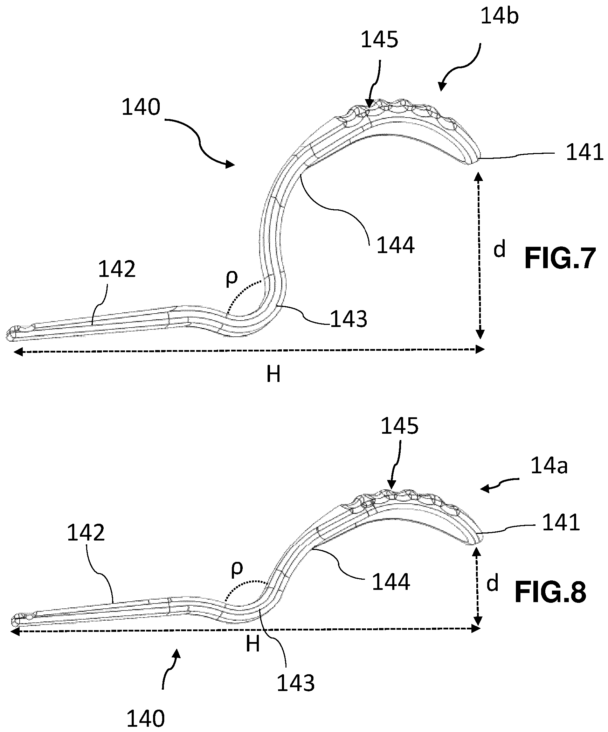

Comme cela est illustré à la figure 7, l'ouverture sommitale 134 s'étend dans un plan sécant de l'ouverture latérale 133, les deux ouvertures 133, 134 étant disposée dans la continuité l'une de l'autre selon un axe sagittal médian de la sonde périnéale 10. Ici, l'axe sagittal médian est disposé dans le plan PSM de la sonde périnéale 10.

Comme illustré aux figures 1 à 4 et 6, la portion évasée 13 comporte au moins une gorge 135, 136, 137. Chaque gorge 135, 136, 137 augmente l'amplitude de la déformation élastique de la portion évasée 13.

En particulier, la portion évasée 13 comporte une gorge longitudinale 135 qui s'étend selon le plan sagittal médian PSM. Ici, la gorge longitudinale 135 divise la portion évasée 13 en deux demi-parois 1321, 1322 selon le plan sagittal médian PSM de la sonde périnéale 10. En pratique, la gorge 135 s'étend au moins partiellement entre la base 131 et le sommet libre 130 de la portion évasée 13. Dans l'exemple illustré à la figure 2, la gorge 135 s'étend sur toute la hauteur de la portion évasée 13 entre la base 131 et le sommet libre 130.

Comme illustré à la figure 2, les deux demi-parois 1321, 1322 sont courbes de manière à former une corolle. En particulier, les demi-parois 1321, 1322 sont courbes de façon convexe par rapport au plan sagittal médian PSM.

Le thérapeute peut alors faire passer la portion évasée 13 de sa position déployée à la position repliée par pincement des demi-parois 1321, 1322 afin de replier la tête de la sonde périnéale 10 selon le plan sagittal médian PSM. Avantageusement, la gorge longitudinale 135 permet d'augmenter l'amplitude du repliement de la tête de la sonde périnéale 10.

Dans l'exemple des figures 1, 4 et 6, le portion évasée 13 comporte au moins une gorge 136, 137 qui s'étend au niveau de la base 131 sur la face extérieure de la paroi latérale 132. La gorge ou les gorge(s) 136, 137 s'étendent transversalement par rapport au plan sagittal médian PSM sur au moins une partie de la circonférence de la base 131 de la portion évasée 13. Ces gorges 136, 137 diminuent l'épaisseur de la paroi latérale 132 afin d'augmenter l'amplitude de repliement de la portion évasée 13. Dans cet exemple, la portion évasée 13 comporte deux gorges transversales 136, 137 qui sont symétriques par rapport au plan sagittal médian PSM. Les deux gorges transversales 136, 137 définissent un plan perpendiculaire du plan sagittal médian PSM.

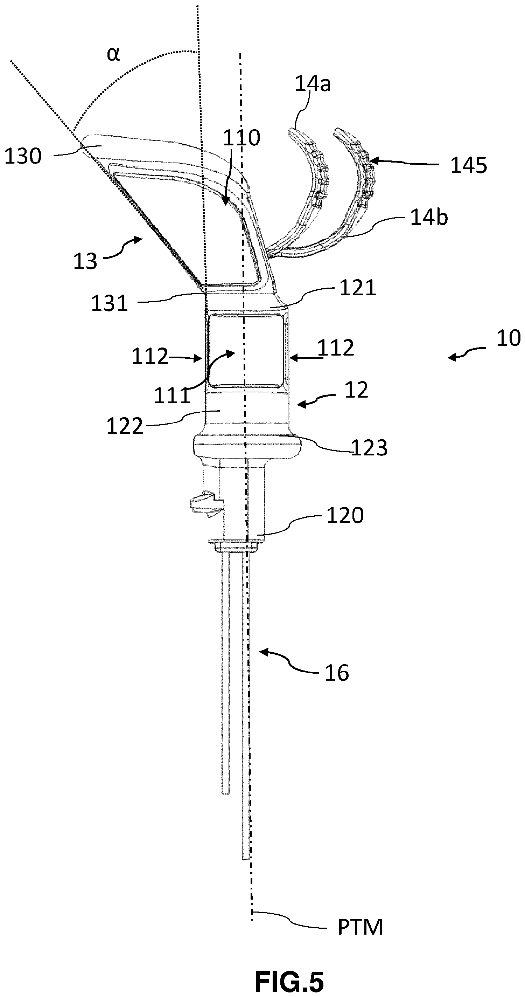

Dans l'exemple de la figure 5, la portion évasée 13 s'étend selon un angle d'inclinaison α par rapport à une paroi latérale 122 de la portion cylindrique 12. Ici, l'angle d'inclinaison α peut être compris entre 7°et 40°l'angle d'inclinaison α peut aussi être compris entre 15° et 30°. L'inclinaison de la portion évasée 13 permet d'adapter la conformation de la sonde périnéale 10 à l'inclinaison antéro-postérieure naturelle du vagin par rapport au plancher pelvien. L'inclinaison de la portion évasée 13 permet d'améliorer le confort de la patiente et le maintien en position de la sonde périnéale 10. De plus, l'inclinaison de la portion évasée 13 joue un rôle de détrompeur afin que le thérapeute introduise la sonde périnéale 10 selon la bonne orientation antéro-postérieure.

Comme illustré aux figures 1 à 3, 5 et 7 à 8, la sonde périnéale 10 comporte un organe de maintien 14a, 14b. L'organe de maintien 14a, 14b est configuré pour prendre appui sur la paroi de l'endocavité afin d'améliorer le maintien en position de la sonde périnéale 10. Dans cet exemple, l'organe de maintien 14a, 14b est mobile entre une position déployée et une position repliée. Dans la position déployée, l'organe de maintien 14a, 14b est saillant du corps de la sonde périnéale 10 et prend appui sur une paroi de l'endocavité du ou de la patiente afin de maintenir la sonde périnéale 10 en position adéquate. A l'inverse, la position repliée de l'organe de maintien 14a, 14b, permet l'introduction de la sonde périnéale 10 dans l'endocavité.

Comme illustré aux figures 1 à 3 et 5, l'organe de maintien 14a, 14b comporte une languette 140 qui s'étend de manière saillante de la portion évasée 13 vers une extrémité libre 141. Ici, la languette 140 est saillante selon le plan sagittal médian PSM de la sonde périnéale 10. La languette 140 est déformable élastiquement de la même façon que la portion évasée 13. A cet effet, la languette 140 peut être constituée dans le même matériau que le corps de la sonde périnéale 10.

La languette 140 est fixée, de manière détachable, de la portion cylindrique 12 de la sonde périnéale 10. Ceci permet d'utiliser plusieurs tailles de languettes 140 en fonction de l'anatomie du ou de la patiente. De manière connue, la profondeur d'un vagin peut varier d'une femme à l'autre en moyenne entre 7 et 15 cm. Le thérapeute peut alors choisir la taille de la languette 140 en fonction de l'anatomie de la patiente.

Les figures 7 et 8 illustrent deux tailles de languettes 140 pour l'organe de maintien 14a, 14b. La figure 5 propose une représentation schématique de la sonde périnéale 10 qui porte fictivement deux organes de maintien 14a, 14b afin de pouvoir apprécier la différence de taille de la languette 140 de chaque organe de maintien 14a, 14b.

Comme illustré aux figures 7 et 8, la languette 140 peut se décomposer en trois segments. A une première extrémité, la languette 140 comporte un segment de fixation 142. Dans cet exemple, le segment de fixation 142 est configuré pour coopérer avec des moyens de réception complémentaires 15 qui sont ménagés sur le corps de la sonde périnéale 10. Les moyens de réception complémentaires 15 sont disposés sur le corps de la sonde périnéale 10 selon le plan PSM.

Dans l'exemple des figures 1 à 3, les moyens de réception complémentaires 15 comportent une fente 150 dont l'ouverture est ménagée sur la face supérieure 124 de la portion cylindrique 12. La fente 150 est disposée selon le plan sagittal médian PSM. De ce fait, la languette 140 peut s'étendre selon ce même plan et dans le même axe de l'ouverture latérale 133 du corps endocavitaire 13. Selon cette disposition, la languette 140 est saillante, en position déployée, de la portion évasée 13 selon un axe antéro-postérieur situé sur le plan PSM. Cette disposition sur le plan PSM permet également de replier, au travers de l'ouverture latérale 133, la languette 140 dans le volume interne de la portion évasée 13. La languette 140 est repliée par déformation élastique dans le volume interne de la portion évasée 13.

Dans l'exemple des figures 7 et 8, le segment de fixation 142 est droit et de section rectangulaire afin de coopérer avec la fente 150. Les dimensions du segment fixation 142 sont complémentaires de celles de la fente 150. Les sections de la fente 150 et du segment de fixation 142 peuvent présenter d'autres formes telles qu'une forme circulaire, hexagonale, triangulaire etc.

Alternativement dans l'exemple de la figure 6, les moyens de réception complémentaires 15 comprennent une encoche 151 ménagée sur la paroi latérale 122 de la portion cylindrique 12. L'encoche 151 est disposée sur le plan PSM. L'encoche 151 est complémentaire de la section du segment de fixation 142 afin de maintenir l'organe de maintien 14a, 14b pendant l'utilisation de la sonde périnéale 10. A ces fins, l'encoche 151 forme une rainure de verrouillage dans laquelle le segment de fixation 142 est inséré et placé en butée du fond de l'encoche 151. Selon le mode de réalisation de la figure 6, la sonde périnéale 10 ne comporte pas d'organes électroniques 112 antéro-postérieurs.

Les moyens de réception complémentaires 15 peuvent également prendre d'autres formes telles que des clips ou tout autre type de moyens de réception mécaniques permettant de fixer de manière amovible l'organe de maintien 14a, 14b. Avantageusement, e caractère détachable de l'organe de maintien 14a, 14b permet de stériliser la sonde périnéale 10 après utilisation ou de choisir une languette 140 adéquate à l'anatomie du ou de la patiente.

Toujours en référence aux figures 7 et 8, la languette 140 comporte un segment de déport 143. Le segment de déport 143 prolonge le segment de fixation 142 en direction de l'extrémité libre 141. Comme illustré aux figures 1 à 3 et 6, le segment de déport 143 fait sailli des moyens de réception 15 de façon à déporter la languette 140 du corps de la sonde périnéale 10. Le segment de déport 143 est configuré pour s'éloigner angulairement du corps de la sonde périnéale 10. Dans cet exemple, le segment de déport 143 est courbé de façon concave par rapport à un axe central du corps de la sonde périnéale. L'axe central s'étend de bas en haut dans le plan PSM et est perpendiculaire à la face supérieure 124 de la portion cylindrique 12.

En référence aux figures 7 et 8, le segment de déport 143 possède un rayon de courbure ρ qui peut être plus ou moins grand selon les différentes tailles de l'organe de maintien 14a, 14b. L'organe de maintien 14a de la figure 8 possède un rayon de courbure ρ supérieur au rayon de courbure ρ de l'organe de maintient 14b de la figure 7.

La languette 140 comporte également un segment d'appui 144. Ici, le segment d'appui 144 s'étend entre le segment de déport 143 et l'extrémité libre 141 de la languette 140. Le segment d'appui 144 est configuré pour prendre appui sur une paroi de l'endocavité lorsque la sonde périnéale 10 est introduite dans l'endocavité. A cet effet, le segment d'appui 144 comporte une surface striée 145. Par exemple, chez un sujet féminin, la surface striée 145 de la languette 140 permet une meilleure adhérence à la paroi vaginale qui présente elle-même des stries. De fait, la surface striée 145 améliore le maintien de la sonde périnéale 10 en position lors d'un protocole de diagnostic ou d'un protocole de rééducation.

Plus particulièrement, la surface striée 145 est disposée sur une portion ovale du segment d'appui 144. La portion ovale possède une courbure de surface à la manière du dos d'une cuillère. Lorsque l'organe de maintien 14a, 14b est en position déployée, la courbure de surface améliore le contact de la surface striée 145 avec la paroi vaginale. Par ailleurs, lorsque l'organe de maintien 14a, 14b est en position repliée, la portion ovale du segment d'appui 144 est disposée dans le volume interne de la portion évasée 13. Dans cette position repliée, la portion ovale du segment d'appui 144 est complémentaire des demi-parois 1321, 1322 en position repliée.

Le segment d'appui 144 et les demi-parois 1321, 1322 forment alors une tête d'insertion dont le profil arrondi facilite l'introduction de la sonde périnéale 10 dans l'endocavité. En conséquence, avant l'introduction de la sonde périnéale 10 dans l'endocavité d'un ou d'une patiente, le thérapeute fait passer l'organe de maintien 14a, 14b en position repliée avant d'exercer une pression sur chaque demi-paroi 1321, 1322 afin de faire passer la portion évasée 13 en position repliée. Une fois la sonde périnéale 10 introduite dans l'endocavité, les propriétés élastiques de la languette 140 et la courbure du segment de déport 143 permettent de bloquer la sonde périnéale 10 en position en exerçant un effort élastique antéro-postérieur contre la paroi de l'endocavité. Cet effort antéro-postérieur permet d'éviter que la sonde périnéale 10 ne soit expulsée de l'endocavité lorsque la ou le patient réaliser un exercice physique de mise en situation en vue d'évaluer l'état fonctionnel et/ou de traiter de manière différenciée et dyssymétrique les plans profonds des plans superficiels du périnée.

Comme illustré aux figures 7 et 8, les dimensions des organes de maintien 14a, 14b peuvent être différentes selon la hauteur H de la languette 140 mais aussi selon la distance d qui correspond au déport de l'extrémité libre 141 par rapport au segment de fixation 142. Plus particulièrement, le rayon de courbure ρ du segment de déport 143 et la longueur du segment d'appui 144 peuvent présenter des dimensions différentes afin de fournir plusieurs organes de maintien 14a, 14b de tailles différentes. Dans l'exemple de la figure 7, le segment d'appui 144 de l'organe de maintien 14b est plus long que le segment d'appui de l'organe de maintien 14a de la figure 8.

Comme illustré aux figures 1 et 3 à 6, les organes électroniques 110, 111, 112 sont disposés sur la portion évasée 13 et/ou sur la portion cylindrique 12.

La sonde périnéale 10 comporte des câbles d'alimentation 16 des organes électroniques 110, 111, 112. Dans cet exemple, la portion cylindrique 12 comporte au moins un passage longitudinal permettant d'intégrer les câbles d'alimentation 16 dans la portion cylindrique 12 de la sonde périnéale 10. En particulier, ledit au moins un passage longitudinal s'étend depuis l'extrémité libre 120 de la portion cylindrique 12 jusqu'à un point de contact avec chaque organe électronique 110, 111, 112. Les câbles d'alimentation 16 relient chaque organe électroniques 110, 111, 112 à une unité de pilotage électronique. En particulier, l'unité de pilotage électronique est capable de gérer chaque organe électronique 110, 111, 112 de façon différenciée. L'unité de pilotage électronique peut consister en un ordinateur ou tout autre terminal électronique configurée pour stocker et exécuter un programme de gestion des organes électroniques 110, 111, 112 de la sonde périnéale 10.

Les connecteurs reliant les câbles d'alimentation 16 aux organes électroniques 110, 111, 112 sont intégrés dans le corps de la sonde périnéale 10 afin de garantir leur isolation. La figure 2 montre deux coussinets 160 à la jonction entre chaque demi-paroi 1321, 1322 et la face supérieure 124 de la portion cylindrique 12. Ces coussinets 160 ménagent respectivement une chambre dans laquelle les contacteurs des câbles électriques 16 sont reliés aux contacteurs des organes électroniques 110, 111, 112.

Alternativement à une alimentation filaire, la sonde périnéale 10 peut comprendre une batterie rechargeable alimentant les organes électroniques 110, 111, 112, des moyens de transmission à distance (par exemple etc.) et une unité de pilotage électronique des organes électroniques 110, 111, 112. Les moyens de transmission à distance peuvent être constitués par un émetteur/récepteur de champs proches de type radio, Bluetooth, Wifi, NFC, RFID etc. En outre, l'unité de pilotage électronique peut comprendre une horloge, un processeur et une mémoire capable de stocker et exécuter des données et des algorithmes tels qu'un programme de diagnostic et/ou un programme de rééducation préalablement chargé dans la mémoire.

Selon un mode de réalisation de l'invention, les organes électroniques 110, 111 sont disposés deux à deux de part et d'autre du plan sagittal médian PSM. De préférence, chaque organe électronique 110, 111 comporte respectivement une électrode EMG. Dans ce document, l'acronyme « EMG » signifie de manière triviale « électromyographique ».

Comme illustré aux figures 4 et 5, les organes électroniques 112 sont disposés sur la portion cylindrique 12. En particulier, les organes électroniques 112 sont disposés deux à deux, de façon antéro-postérieure, de part et d'autre d'un plan transversal médian PTM de la sonde périnéale 10. Selon cette configuration, chaque organe électronique 112 comporte un capteur de force. Le plan transversal médian PTM représenté à la figure 5 définit un plan de symétrie longitudinal selon une orientation transversale de la sonde périnéale 10.

La sonde périnéale 10 peut comporter uniquement les organes électroniques 110, 111 équipés d'électrodes EMG ou uniquement les organes électroniques 112 constitués respectivement par un capteur de force. Toutefois, la sonde périnéale 10 peut également associer tous les organes électroniques 110, 111, 112 (voir figure 4). Dans ce cas, la sonde périnéale 10 peut fonctionner selon deux modes : un mode EMG, dans lequel, seuls les organes électroniques 110, 111 sont actifs, et un mode de mesure de force, dans lequel, seul les organes électroniques 112 sont actifs.

Comme illustré aux figures 3, 4 et 6 la portion évasée 13 comprend deux organes électroniques 110 disposés de part et d'autre d'un plan de symétrie de la sonde périnéale 10. Dans cet exemple, les deux organes électroniques 110 sont disposés de part et d'autre du plan PSM.

Comme illustré aux figures 4 et 5, chaque organe électronique 110 de la portion évasée 13 peut s'étendre sur plus de 50% de la surface de la face extérieure chaque demi-paroi 1321, 1322. De préférence, chaque organe électronique 110 peut s'étendre sur plus de 70% de la surface de la face extérieure chaque demi-paroi 1321, 1322. Encore de préférence, chaque organe électronique 110 peut s'étendre sur plus de 90% de la surface de la face extérieure chaque demi-paroi 1321, 1322. Lorsque l'organe électronique 110 comporte une électrode EMG, l'électrode s'étend sur la totalité de la surface de l'organe électronique 110. Or, une grande surface d'électrode permet, de répartir plus largement sur l'anatomie du ou de la patiente, l'intensité du courant électrique par exemple dans le cadre d'un programme d'électrostimulation.

En effet, la Norme 60601-2-10 en ce qui concerne les électrodes d'électrostimulation préconise que la densité de courant d'une électrode ne doit pas dépasser un seuil maximal de 2 mA/cm². Or, pour obtenir une réponse électro-induite il faut fournir au moins 30mA. De fait, augmenter la surface de l'électrode tel que cela est exposé précédemment permet de respecter la norme tout en obtenant une réponse électro-induite satisfaisante.

Comme illustré à la figure 5, chaque organe électronique 110 présente une forme trapézoïdale dont les angles supérieurs sont arrondis. Cette forme permet de suivre la conformation de chaque demi-paroi 1321, 1322 et plus largement de la portion évasée 13. La surface de chaque organe électronique 110 est par conséquent courbe. Chaque organe électronique 110 s'étend vers le haut depuis la base 131 jusqu'au sommet libre 130 de la portion évasée 13.

Comme illustré aux figures 3, 4 et 6, la portion cylindrique 13 comprend également deux organes électroniques 111 disposés de part et d'autre d'un plan PSM de la sonde périnéale 10. Plus particulièrement, les organes électroniques 111 sont disposés en partie haute du premier tronçon de la portion cylindrique 12. Ladite partie haute se situe à proximité de la seconde extrémité 121 de la portion cylindrique 12.

La figure 4 illustre schématiquement une sonde périnéale 10 en position dans une cavité vaginale selon une vue postérieure d'une coupe transversale de la cavité vaginale.

Selon cette représentation, les demi-parois 1321, 1322 et les organes électroniques 110 sont au contact des parois 200 de la cavité vaginale. De ce fait, les organes électroniques 110 sont disposés au regard des muscles du plan profond du périnée qui ceinturent la cavité vaginale. Les muscles du plan profond du périnée comportent notamment les muscles pubo-vaginaux ou pubo-coccygiens mais aussi les muscles pubo-rectaux. Ce sont des muscles pairs qui ceinturent la cavité vaginale et rectale. Les muscles ilio coccygiens et ischio coccygiens qui forment deux coupoles périnéales. Les coupoles périnéales se trouvent physiologiquement en pré-tension musculaire et disposent d'une énergie potentielle d'amortissement de la pression intraabdominale. Ici, les muscles ilio coccygiens et ischio coccygiens 210 sont représentés au contact de la paroi vaginale 200. Le plan profond du périnée comporte également les muscles pubo-rectaux. De fait, lorsque la sonde périnéale 10 est insérée dans la cavité anale, les organes électroniques 110 sont positionnés au regard des muscles ilio coccygiens et ischio coccygiens.

Comme illustré à la figure 4, lorsque la sonde périnéales 10 est en position dans la cavité vaginale, les organes électroniques 111 de la portion cylindrique 13 sont également placés au contact de la paroi vaginale 200 à proximité de l'ouverture du vagin. Les organes électroniques 111 sont alors disposés en vis-à-vis des muscles du plan superficiels du périnée. Les muscles superficiels du périnée comportent notamment les muscles bulbo-spongieux et ischiocarverneux. Ce sont également des muscles pairs qui ceinturent l'extrémité basse du vagin et de l'urètre. D'un point de vue fonctionnel, la contraction des muscles bulbo-spongieux et ischiocarverneux renforcent la fermeture des sphincters urétraux. Le plan superficiel du périnée comporte également le sphincter externe anal. De fait, lorsque la sonde périnéale 10 est insérée dans la cavité anale, les organes électroniques 111 sont positionnés en vis-à-vis du sphincter externe anal.

En référence aux figures 4 et 5, les organes électroniques 112, qui sont constitués par des capteurs de force, sont disposés sur la partie haute de la portion cylindrique 12. De fait, lorsque la sonde périnéale 10 est insérée dans l'endocavité, les capteurs de force sont positionnés au niveau du plan superficiel du périnée. Il est ainsi possible de mesurer les forces qui s'exercent, selon un axe antéro-postérieur, sur l'extrémité basse de l'endocavité. Par exemple, il est possible de déduire de ces mesures l'état fonctionnel des muscles pubo-vaginaux, bulbo-spongieux et ischiocarverneux qui cravatent le vagin et l'urètre.

Pour des questions d'hygiène, lorsque l'on utilise les capteurs de force, la sonde périnéale 10 est insérée dans une enveloppe de type préservatif avant d'être introduite dans l'endocavité d'un ou d'une patiente. Néanmoins, dans tous les cas, il est recommandé de décontaminer la sonde périnéale 10 après chaque utilisation.

De préférence, la sonde périnéale 10 comporte deux organes électroniques 110 disposés sur la portion évasée 13 et deux organes électroniques 111 disposés sur la portion cylindrique 12 du corps de la sonde périnéale 10.

Selon cette configuration on peut évaluer, au travers de leur activité EMG, l'état fonctionnel et/ou rééduquer les muscles des plans profonds et superficiels du périnée. De fait, la sonde périnéale 10 est configurée pour interagir de manière différenciée avec des structures anatomiques disposées de part et d'autre du plan sagittal médian de l'endocavité du ou de la patiente.

Dans l'exemple de la figure 4, la disposition des organes électroniques 110, 111 de part et d'autre du plan sagittal médian PSM de la sonde périnéal 10 permet de traiter de manière différenciée, les faisceaux des muscles ilio coccygiens et ischio coccygiens et/ou pubo-vaginaux, ischiocaverneux et bubo-spongieux, qui se situent de part et d'autre du vagin. La sonde périnéale 10 permet donc de diagnostiquer et/ou de traiter un état fonctionnel dyssymétrique de ces muscles.

De plus, la sonde périnéale 10 est configurée pour interagir de manière différenciée avec des structures anatomiques qui ceinturent l'endocavité du/de la patient(e) disposées selon deux plans anatomiques distincts. En référence à la figure 4, la disposition d'organes électroniques 110, 111 sur la portion cylindrique 12 et sur la portion évasée 13 permet de traiter de manière indépendante les muscles des plans superficiels et profonds du périnée.

La possibilité de traiter de manière différenciée les muscles situés de part et d'autre du vagin selon deux plans anatomiques différents est également rendue possible par le fait que chaque organe électronique 110, 111 comporte son propre câble électrique 16 qui le relie de manière indépendante à une unité de pilotage.

Comme cela a été indiqué en introduction de ce document, le phénomène de continence est un phénomène complexe qui implique plusieurs groupes de muscles tant par leur tonicité que leur réactivité ou que la qualité des réflexes myotatiques et conditionnés.

Le premier groupe de muscles qui est impliqué correspond aux sphincters urétraux et anaux qui ceinturent respectivement l'urètre et le canal anal. Sous l'effet de la contraction respective de ces muscles, l'urètre et le canal anal se ferment.

Les muscles du périnée constituent un deuxième groupe de muscles qui participe au mécanisme de la continence. Le plan superficiel des muscles du périnée entoure l'extrémité inférieure de l'urètre, du vagin et du canal anal. Alors que les muscles profonds du périnée forment deux coupoles périnéales qui sont en pré-tension musculaire et disposent d'une énergie potentielle d'amortissement de la pression intraabdominale. Les muscles profonds entourent également la cavité vaginale selon un plan anatomique plus profond.

Comme cela a été évoqué, les muscles des plans profonds et des plans superficiels du périnée n'exercent pas le même rôle dans le mécanisme de la continence. Les muscles profonds du périnée ont une fonction d'amortisseur de l'augmentation de la pression intraabdominale qui est un phénomène déclencheur d'une situation potentiel de fuite urinaire ou fécale. Les muscles superficiels du périnée participent quant à eux la fermeture de l'urètre ou du canal anal.

Lors d'un examen clinique au cours duquel la sonde périnéale 10 est introduite dans l'endocavité d'un ou d'une patiente avant que cette dernière réalise des exercices qui entrainent habituellement une fuite urinaire ou fécale : un effort de toux, des genou-flexions, une inclinaison du buste vers l'avant, le transport de charges etc.

Lors de ces exercices, la sonde périnéale 10 permet de mesurer, au travers de l'activité EMG, le tonus de base des muscles des plans superficiels et profonds du périnée, le réflexe d'anticipation périnéal, l'état du tonus musculaire après le réflexe périnéal, le réflexe myotatique, mais aussi la réponse des fibres rapides musculaires initiée par ce réflexe.

Par ailleurs, au travers du suivi des forces qui s'appliquent sur les capteurs de force, au travers de la paroi de l'endocavité, on peut également évaluer le tonus de base des plans profonds et superficiels du périnée, le réflexe d'anticipation périnéal, la réponse des fibres rapides, le timing du réflexe myotatique (est-il présent ? ou retardé ?) etc.

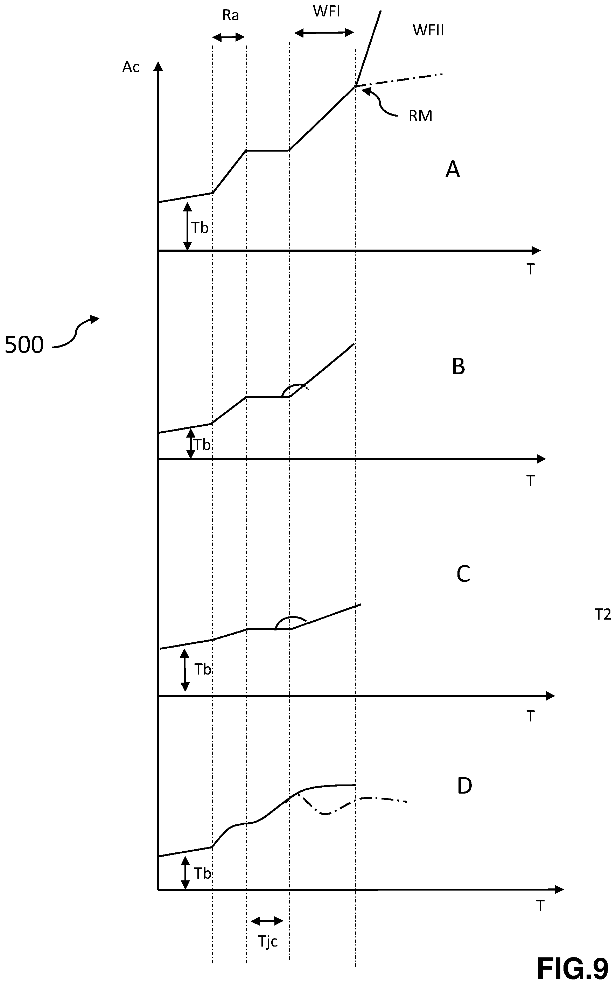

Le diagramme 500 de la figure 9 illustre de manière schématique quatre enregistrements A, B, C, D de l'activité Ac neuromusculaire mesurée, par les organes électroniques 110, 111, 112 de la sonde périnéale 10 conforme de l'invention, en fonction du temps T.

L'enregistrement A correspond aux données mesurées par un capteur de force postérieur de l'organe électronique 112. Ce dernier est placé au niveau du plan superficiel du périnée. En premier lieu, l'on mesure le tonus de base Tb de l'endocavité et des muscles superficiels du périnée. Alors que la patiente s'apprête à faire un mouvement entrainant une augmentation de la pression intraabdominale, on distingue sur le diagramme 500 une augmentation de l'activité neuromusculaire qui correspond au réflexe d'anticipation périnéale Ra lequel active les fibres I. Les données mesurées à la suite du réflexe d'anticipation Ra correspondent au tonus du tissu conjonctif Tjc majoré par l'augmentation de l'activité neuromusculaire (fibre I). En principe, le tonus d'un tissu conjonctif fonctionnel est remarquable par un tracé sous forme de plateau tel qu'illustré pour l'enregistrement A.

En réponse à l'étirement musculaire généré par l'augmentation de la pression intraabdominale, le recrutement des fibres musculaires de type I ou fibres lentes augment progressivement la contraction musculaire et la pression de clôture (urétrale et anale). Cette phase est notée WFI sur l'enregistrement A. Lorsque l'étirement atteint un seuil déterminé, le réflexe myotatique RM déclenche la réponse WFII des fibres musculaires de type II ou fibres rapides. L'enregistrement A schématise une ligne continue dont la pente de la courbe augmente manière substantielle après le réflexe myotatique RM et une courbe en pointillé pour laquelle la pente de la courbe diminue. Or, le tracé en pointillé de la courbe de l'enregistrement A est caractéristique d'un dysfonctionnement des fibres rapides ou du reflexe myotatique.

Les enregistrements B et C du diagramme 500 correspondent respectivement aux données mesurées par les capteurs EMG des organes électroniques 110 de la portion évasée 13. Ces enregistrements correspondent donc à l'activité des muscles périnéaux de part et d'autre du plan PSM. On note sur le tracé de l'enregistrement C une hypertonicité du tonus de base Tb et un profil de courbe moins marqué que celui de l'enregistrement B. De tels enregistrements peuvent révéler une dissymétrie fonctionnelle des coupoles périnéales. Cette dissymétrie peut se retrouver chez la femme enceinte en fonction du positionnement de la tête du bébé. Toutefois, l'hypertonie dyssymétrique peut également être causée par un traumatisme obstétrical, physique ou psychologique.

L'enregistrement D du diagramme 500 correspond aux données mesurées par le capteur de force antérieur de l'organe électronique 112. Ce dernier est placé au niveau du plan superficiel du périnée. Sur cet enregistrement, le tracé en pointillé correspond à une dégradation de la qualité du tissu conjonctif du du périnée.

La sonde périnéale 10 permet d'établir des diagnostics différenciés des différents muscles qui constituent les plans superficiels et profonds du périnée. En particulier, il est possible d'évaluer de manière différenciée l'état fonctionnel des deux faisceaux pairs de muscles ilio coccygiens et ischio coccygiens et/ou pubo-vaginaux et pubo rectaux (plans profonds), des muscles bulbo-spongieux et des muscles ischiocaverneux (plans superficiels).

La sonde périnéale 10 permet d'établir de manière précise le dysfonctionnement : hypertonie ou hypotonie du tonus de base et/ou du tonus du tissu conjonctif, dysfonctionnement des fibres rapides et/ou des fibres lentes, absence ou retard du réflexe d'anticipation, absence ou retard du réflexe myotatique etc. Les données mesurées peuvent être interprétées directement ou par comparaison avec une base de données de référence.

Un fois le diagnostic établi, la sonde périnéale 10 permet d'effectuer un protocole de rééducation fonctionnel qui peut être ciblé sur les structures anatomiques qui présentent un trouble de leur état fonctionnel. Selon le trouble identifié, le thérapeute peut appliquer au travers des électrodes EMG des programmes d'électrostimulation adaptés pour traiter le ou les dysfonctionnements diagnostiqués.

## Revendications

1. Sonde périnéale (10) comprenant un corps de sonde qui comporte :
- une portion cylindrique (12) configurée pour être positionnée au niveau de l'ouverture de l'endocavité vaginale ou anale d'un(e) patient(e),
- une portion évasée (13) qui s'étend depuis une extrémité (121) de la portion cylindrique (12), la portion évasée (13) s'étend de manière évasée depuis la portion cylindrique (12) jusqu'à un sommet libre (130), la portion évasée (13) étant élastiquement déformable entre une position repliée et une position déployée de façon à prendre appui sur les parois de l'endocavité, et
- des organes électroniques (110, 111, 112) configurés pour interagir avec des muscles du périnée d'un(e) patient(e), les organes électroniques (110, 111, 112) étant disposés sur la portion évasée (13) et/ou sur la portion cylindrique (12),
**caractérisée en ce que** la portion évasée (13) comporte, d'une part, au moins une paroi latérale (132) qui présente une face interne (1320) délimitant un volume interne conique de la portion évasée (13), et d'autre part, au moins une gorge longitudinale (135) qui s'étend selon un plan de symétrie de la sonde périnéale (10),

2. Sonde périnéale (10) selon la revendication 1, dans laquelle, la gorge longitudinale (135) s'étend selon un plan de symétrie sagittal médian (PSM) de la sonde périnéale (10) et divise ladite au moins une paroi latérale (132) en deux demi-parois (1321, 1322).

3. Sonde périnéale (10) selon la revendication 2, dans laquelle, la portion évasée (13) comporte au moins une gorge (136, 137) qui s'étend transversalement par rapport au plan sagittal médian (PSM) de la sonde périnéale (10), la gorge (136, 137) s'étendant sur la face extérieure de la paroi latérale (132) au niveau d'une base (131) disposée à la jonction entre la portion évasée (13) et la portion cylindrique (12).

4. Sonde périnéale (10) selon l'une des revendications 1 à 3, dans laquelle, la portion évasée (13) comporte au moins une ouverture (133, 134) disposée selon un plan de symétrie de la sonde périnéale (10).

5. Sonde périnéale (10) selon la revendication 4, dans laquelle, la portion évasée (13) comporte :
- une ouverture latérale (133) disposée selon un plan de symétrie de la sonde périnéale (10), et
- une ouverture sommitale (134) disposée au niveau du sommet libre (130) de la portion évasée (13).

6. Sonde périnéale (10) selon l'une des revendications 1 à 5, qui comporte, un organe de maintien (14a, 14b) qui est mobile entre une position déployée et une position repliée, en position déployée, l'organe de maintien (14a, 14b) permet de maintenir la portion évasée (13) en position dans l'endocavité du ou de la patient(e), et, en position repliée, l'organe de maintien (14a, 14b) permet l'introduction de la sonde périnéale (10) dans l'endocavité.

7. Sonde périnéale (10) selon la revendication 6, dans laquelle, l'organe de maintien (14a, 14b) comporte une languette (140) qui s'étend de manière saillante de la portion évasée (13) vers une extrémité libre (141), la languette (140) étant saillante selon un plan de symétrie de la sonde périnéale (10).

8. Sonde périnéale (10) selon la revendication 7, dans laquelle, la languette (140) comporte à son extrémité libre (141) une surface striée (145).

9. Sonde périnéale (10) selon l'une des revendications 6 à 8, dans laquelle, l'organe de maintien (14a, 14b) est attaché à la portion cylindrique (12) de manière détachable.

10. Sonde périnéale (10) selon l'une des revendications 7 à 9, dans laquelle, la languette (140) est saillante, en position déployée, de la portion évasée (13) selon un axe antéro-postérieur situé sur le plan (PSM).

11. Sonde périnéale (10) selon l'une des revendications 1 à 10, dans laquelle, la portion évasée (13) s'étend selon un angle d'inclinaison α par rapport à une paroi latérale (122) de la portion cylindrique (12), l'angle d'inclinaison α étant compris entre 7°et 40°.

12. Sonde périnéale (10) selon l'une des revendications 1 à 11, dans laquelle, la portion évasée (13) comprend deux organes électroniques (110) disposés de part et d'autre d'un plan de symétrie de la sonde périnéale (10).

13. Sonde périnéale (10) selon l'une des revendications 1 à 12., dans laquelle, la portion cylindrique (12) comprend deux organes électroniques (111, 112) disposés de part et d'autre d'un plan de symétrie de la sonde périnéale (10).

14. Sonde périnéale (10) selon les revendications 10 et 13, dans laquelle, la portion cylindrique (12) et la portion évasée (13) comportent respectivement des organes électroniques (110, 111) constitués par des électrodes EMG, les organes électroniques (110, 111) étant disposées de part et d'autre d'un plan sagittal médian PSM de la sonde périnéale (10).

15. Sonde périnéale selon l'une des revendications 1 à 14, dans laquelle, les organes électroniques (112) comportent deux capteurs de force, les organes électroniques (112) disposés de façon antéro-postérieure de part et d'autre d'un plan transversal médian PTM de la sonde périnéale (10).

## Patentansprüche

1. Perinealsonde (10), umfassend einen Sondenkörper, der Folgendes aufweist:
- einen zylindrischen Abschnitt (12), der konfiguriert ist, um auf Ebene der Öffnung der vaginalen oder analen Endokavität eines Patienten positioniert zu werden,
- einen aufgeweiteten Abschnitt (13), der sich von einem Ende (121) des zylindrischen Abschnitts (12) erstreckt, wobei sich der aufgeweitete Abschnitt (13) aufgeweitet von dem zylindrischen Abschnitt (12) zu einem freien Scheitelpunkt (130) erstreckt, wobei der aufgeweitete Abschnitt (13) zwischen einer gefalteten Position und einer entfalteten Position elastisch verformbar ist, um an den Wänden der Endokavität in Anlage zu kommen, und
- elektronische Elemente (110, 111, 112), die konfiguriert sind, um mit Muskeln des Perineums eines Patienten zu interagieren, wobei die elektronischen Elemente (110, 111, 112) an dem aufgeweiteten Abschnitt (13) und/oder an dem zylindrischen Abschnitt (12) angeordnet sind,
**dadurch gekennzeichnet, dass** der aufgeweitete Abschnitt (13) einerseits mindestens eine Seitenwand (132), die eine Innenfläche (1320) aufweist, die ein konisches Innenvolumen des aufgeweiteten Abschnitts (13) begrenzt, und andererseits mindestens eine Längsnut (135), die sich entlang einer Symmetrieebene der Perinealsonde (10) erstreckt, umfasst,

2. Perinealsonde (10) nach Anspruch 1, wobei sich die Längsnut (135) entlang einer mittleren sagittalen Symmetrieebene (MSP) der Perinealsonde (10) erstreckt und die mindestens eine Seitenwand (132) in zwei Halbwände (1321, 1322) unterteilt.

3. Perinealsonde (10) nach Anspruch 2, wobei der aufgeweitete Abschnitt (13) mindestens eine Nut (136, 137) aufweist, die sich quer in Bezug auf die mittlere Sagittalebene (MSP) der Perinealsonde (10) erstreckt, wobei sich die Nut (136, 137) an der Außenseite der Seitenwand (132) auf Ebene einer Basis (131) erstreckt, die an der Verbindung zwischen dem aufgeweiteten Abschnitt (13) und dem zylindrischen Abschnitt (12) angeordnet ist.

4. Perinealsonde (10) nach einem der Ansprüche 1 bis 3, wobei der aufgeweitete Abschnitt (13) mindestens eine Öffnung (133, 134) aufweist, die entlang einer Symmetrieebene der Perinealsonde (10) angeordnet ist.

5. Perinealsonde (10) nach Anspruch 4, wobei der aufgeweitete Abschnitt (13) Folgendes aufweist:
- eine seitliche Öffnung (133), die entlang einer Symmetrieebene der Perinealsonde (10) angeordnet ist, und
- eine Scheitelöffnung (134), die auf Ebene der freien Spitze (130) des aufgeweiteten Abschnitts (13) angeordnet ist.

6. Perinealsonde (10) nach einem der Ansprüche 1 bis 5, die ein Halteelement (14a, 14b) aufweist, das zwischen einer entfalteten Position und einer gefalteten Position beweglich ist, wobei das Halteelement (14a, 14b) in der entfalteten Position ermöglicht, den aufgeweiteten Abschnitt (13) in der Endokavität des Patienten oder der Patientin in Position zu halten, und das Halteelement (14a, 14b) in der eingeklappten Position das Einführen der Perinealsonde (10) in die Endokavität ermöglicht.

7. Perinealsonde (10) nach Anspruch 6, wobei das Halteelement (14a, 14b) eine Zunge (140) umfasst, die sich hervorstehend von dem aufgeweiteten Abschnitt (13) zu einem freien Ende (141) erstreckt, wobei die Zunge (140) entlang einer Symmetrieebene der Perinealsonde (10) hervorstehend ist.

8. Perinealsonde (10) nach Anspruch 7, wobei die Zunge (140) an ihrem freien Ende (141) eine gerillte Oberfläche (145) aufweist.

9. Perinealsonde (10) nach einem der Ansprüche 6 bis 8, wobei das Halteelement (14a, 14b) lösbar an dem zylindrischen Abschnitt (12) angebracht ist.

10. Perinealsonde (10) nach einem der Ansprüche 7 bis 9, wobei die Zunge (140) in der entfalteten Position von dem aufgeweiteten Abschnitt (13) entlang einer anterior-posterioren Achse, die in der (PSM)-Ebene liegt, hervorsteht.

11. Perinealsonde (10) nach einem der Ansprüche 1 bis 10, wobei sich der aufgeweitete Abschnitt (13) in einem Neigungswinkel α in Bezug auf eine Seitenwand (122) des zylindrischen Abschnitts (12) erstreckt, wobei der Neigungswinkel α zwischen 7°und 40° liegt.

12. Perinealsonde (10) nach einem der Ansprüche 1 bis 11, wobei der aufgeweitete Abschnitt (13) zwei elektronische Elemente (110) umfasst, die auf beiden Seiten einer Symmetrieebene der Perinealsonde (10) angeordnet sind.

13. Perinealsonde (10) nach einem der Ansprüche 1 bis 12, wobei der zylindrische Abschnitt (12) zwei elektronische Elemente (111, 112) umfasst, die auf beiden Seiten einer Symmetrieebene der Perinealsonde (10) angeordnet sind.

14. Perinealsonde (10) nach den Ansprüchen 10 und 13, wobei der zylindrische Abschnitt (12) und der aufgeweitete Abschnitt (13) jeweils elektronische Elemente (110, 111) aufweisen, die aus EMG-Elektroden bestehen, wobei die elektronischen Elemente (110, 111) auf beiden Seiten einer mittleren Sagittalebene PSM der Perinealsonde (10) angeordnet sind.

15. Perinealsonde nach einem der Ansprüche 1 bis 14, wobei die elektronischen Elemente (112) zwei Kraftsensoren umfassen, wobei die elektronischen Elemente (112) anterior-posterior auf beiden Seiten einer transversalen Mittelebene PTM der Perinealsonde (10) angeordnet sind.

## Claims

1. Perineal probe (10) comprising a probe body which includes:
- a cylindrical portion (12) configured to be positioned at the opening of the vaginal or anal endocavity of a patient,
- a flared portion (13) which extends from one end (121) of the cylindrical portion (12), the flared portion (13) extends in a flared manner from the cylindrical portion (12) to a free upper end (130), the flared portion (13) being elastically deformable between a folded position and a deployed position so as to rest on the walls of the endocavity, and
- electronic organs (110, 111, 112) configured to interact with the muscles of the perineum of a patient, the electronic organs (110, 111, 112) being disposed on the flared portion (13) and/or on the cylindrical portion (12), **characterized in that** the flared portion (13) includes, on the one hand, at least one side wall (132) which has an internal face (1320) delimiting a conical internal volume of the flared portion (13), and, on the other hand, at least one longitudinal groove (135) which extends in a plane of symmetry of the perineal probe (10).

2. The perineal probe (10) as claimed in claim 1 in which the longitudinal groove (135) extends in a median sagittal plane of symmetry (PSM) of the perineal probe (10) and divides said at least one lateral wall (132) into two half-walls (1321, 1322).

3. The perineal probe (10) as claimed in claim 2 in which the flared portion (13) includes at least one groove (136, 137) that extends transversely relative to the median sagittal plane (PSM) of the perineal probe (10), the groove (136, 137) extending on the exterior face of the lateral wall (132) at the level of a base (131) disposed at the junction between the flared portion (13) and the cylindrical portion (12).

4. The perineal probe (10) as claimed in any one of claims 1 to 3 in which the flared portion (13) includes at least one opening (133, 134) disposed in a plane of symmetry of the perineal probe (10).

5. The perineal probe (10) as claimed in claim 4 in which the flared portion (13) includes:
- a lateral opening (133) disposed in a plane of symmetry of the perineal probe (10), and
- a top opening (134) disposed at the level of the free upper end (130) of the flared portion (13).

6. The perineal probe (10) as claimed in any one of claims 1 to 5, including a retaining member (14a, 14b) that is mobile between a deployed position and a folded position and in which, in the deployed position, the retaining member (14a, 14b) enables the flared portion (13) to be retained in position in the endocavity of the patient and, in the folded position, the retaining member (14a, 14b) enables insertion of the perineal probe (10) into the endocavity.

7. The perineal probe (10) as claimed in claim 6 in which the retaining member (14a, 14b) includes a tongue (140) that projects from the flared portion (13) toward a free end (141), the tongue (140) projecting in a plane of symmetry of the perineal probe (10).

8. The perineal probe (10) as claimed in claim 7 in which the tongue (140) has a striated surface (145) at its free end (141).

9. The perineal probe (10) as claimed in any one of claims 6 to 8 in which the retaining member (14a, 14b) is attached to the cylindrical portion (12) in a detachable manner.

10. The perineal probe (10) as claimed in any one of claims 7 to 9 in which in the deployed position the tongue (140) projects from the flared portion (13) along an anterior-posterior axis in the median sagittal plane (PSM).

11. The perineal probe (10) as claimed in any one of claims 1 to 10 in which the flared portion (13) extends at an angle of inclination α relative to a lateral wall (122) of the cylindrical portion (12), the angle of inclination α being between 7°and 40° inclusive.

12. The perineal probe (10) as claimed in any one of claims 1 to 11 in which the flared portion (13) includes two electronic members (110) disposed on either side of a plane of symmetry of the perineal probe (10).

13. The perineal probe (10) as claimed in any one of claims 1 to 12 in which the cylindrical portion (12) includes electronic members (111, 112) disposed on either side of a plane of symmetry of the perineal probe (10).

14. The perineal probe (10) as claimed in claims 10 and 13 in which the cylindrical portion (12) and the flared portion (13) include respective electronic members (110, 111) consisting of EMG electrodes, the electronic members (110, 111) being disposed on either side of a median sagittal plane PSM of the perineal probe (10).

15. The perineal probe as claimed in any one of claims 1 to 14 in which the electronic members (112) disposed in an anterior-posterior manner on either side of a median transverse plane PTM of the perineal probe (10) include two force sensors.
